# EUROPÄISCHE PATENTANMELDUNG

(11) **EP 1 081 479 A1**
(43) Veröffentlichungstag der Anmeldung: **07.03.2001**
(21) Anmeldenummer: 00117731.0
(22) Anmeldetag: 17.08.2000
(51) Int. Cl.: G01K 13/00, A61B 10/00

(54) **Medizinisches Thermometer**

(30) Priorität: 03.09.1999 DE 19942089
(71) Anmelder: Geratherm Medical AG, 98716 Geschwenda (DE)
(72) Erfinder: Kuhn, Jens, Dr., 98693 Ilmenau (DE)
(74) Vertreter: Strehl Schübel-Hopf & Partner

(57) **Zusammenfassung**

Die Erfindung betrifft ein medizinisches Thermometer in einer kleinen, auch für Kinder tragbaren Bauform mit Datenspeicher **18** und Schnittstelle **21**, mit dem sowohl kontinuierlich überwachende als auch diskontinuierlich zyklische Messungen als Maximumthermometer an medizinisch anerkannten Körpertemperatur-Meßstellen komfortabel ausgeführt werden können. Durch Einstellen der Speicherrate wird festlegt, ob die Temperatur der Meßstelle kontinuierlich überwacht wird oder zyklische bzw. Einzelmessungen ausführt werden. Zum Wechseln der Betriebsart sind keine zusätzlichen Taster oder Schalter notwendig, die an einem kleinen kompakten Gehäuse **14** nur schwer zu plazieren wären.

## Beschreibung

Kleine Kinder und Säuglinge erkranken relativ leicht an Erkältungen und verschiedenen Infektionskrankheiten, z.B. an den sogenannten Kinderkrankheiten (Masern, Windpocken, Röteln, Scharlach usw.). Diese Krankheiten verursachen bei den Eltern nicht unerheblichen Streß nicht zuletzt dadurch, daß regelmäßig die Körpertemperatur des Kindes überprüft werden muß, um sofort zu erkennen, ob drastische Veränderungen eingetreten sind. Dazu müssen die Eltern in der Nacht sowohl ihren eigenen Schlaf als auch den des Kindes unterbrechen. Das ist für alle Beteiligten wenig erfreulich. Wünschenswert wäre deshalb ein Thermometer, das diese Überwachung selbsttätig vornimmt, kontinuierlich die Körpertemperatur ermittelt und bei kritischen Veränderungen Alarm auslöst.

Oft ist es für die Eltern auch nicht leicht zu entscheiden, inwieweit eingetretene Temperaturerhöhungen eventuell schon gefährlich für das Kind sind. Es wäre deshalb äußerst vorteilhaft, wenn die ermittelten Temperaturkurven auf einfache Weise, z.B. per e-Mail, dem Hausarzt übermittelt werden könnten, damit dieser die Meßergebnisse fachkundig begutachten und entsprechende Maßnahmen veranlassen kann.

Gefährlich kann Fieber werden, wenn es sogenannte Fieberkrämpfe auslöst, die mit einem Bewußtseinsverlust verbunden sein können. Ausgelöst werden diese Krämpfe durch einen plötzlichen Fieberanstieg. Auftreten kann ein solcher Krampfanfall vor allem bei Kindern ab dem 6. Monat bis zum 5. Lebensjahr. Etwa 3 bis 4 % der Kinder in dieser Altersgruppe sind betroffen. Das Risiko, später afibrile Anfälle, also eine Epilepsie, zu entwickeln, steigt mit zunehmender Dauer des Anfalls. Daher ist die optimale Therapie im akuten Anfall besonders wichtig. Ein Anfall, bei dem nicht innerhalb von wenigen Minuten krampflösende Maßnahmen eingeleitet werden, stellt einen echten Notfall dar. Um das Risiko des Auftretens und vor allem der Wiederholung von Fieberkrämpfen zu verringern, werden frühzeitige fiebersenkende Maßnahmen ab einer Körpertemperatur von 38,5 °C empfohlen. Es wird also auch in diesem Fall ein kontinuierlich messendes Thermometer benötigt, das bei Überschreiten einer Grenztemperatur Alarm auslöst, um ein schnelles Eingreifen zu ermöglichen. Da ein großer Teil der gefährdeten Kinder mobil ist, muß dieses Thermometer für Kinder problemlos tragbar sein.

Bei älteren, insbesondere hilflosen Personen kann es z.B. durch zu geringe Nahrungsaufnahme oder chronische Infektionen zu Auszehrungen (Kachexie) kommen, die ihrerseits lebensbedrohliche Unterkühlungen (Hypothermie) verursachen können. Unter ca. 30°C Körpertemperatur kann es zu einem Bewußtseinsverlust kommen, der es dem Betroffenen unmöglich macht, sich selbst zu helfen. Mit Hilfe eines kontinuierlich messenden Thermometers mit einer Alarmfunktion bei Unterschreiten einer Grenztemperatur kann bei Risikopersonen in akuten Fällen schnell Hilfe herbeigerufen werden.

Eine relativ zuverlässige, medikamentenlose Methode der natürlichen Kontrazeption ist die Temperaturmethode, bei der die Frau durch Eigenbeobachtung der Basaltemperatur die fruchtbaren Tage im Menstruationszyklus bestimmt. Die Aufwachtemperatur ist auch ein wichtiger Parameter in Diagnose und Therapie von Zyklusstörungen. Zur Aufzeichnung und Auswertung der Basaltemperatur benötigt man ein Thermometer, das als Maximumthermometer arbeitet. Dabei wird die Temperatur der Meßstelle so lange ermittelt, bis ein Grenzwert für den Anstiegsgradienten erreicht bzw. unterschritten wird. Bei dieser Art von Thermometer wird stets nur der größte bei der einzelnen Messung gefundene Temperaturwert angezeigt, das namensgebende Maximum. Als Zyklo-Thermometer ist ein Maximumthermometer einsetzbar, das das Temperaturmaximum zusammen mit der Uhrzeit und einer Tageszahl speichert und bei Bedarf diese Informationen auf einem Display anzeigt bzw. an einen PC übertragen kann.

Für die kontinuierliche Überwachung der Körpertemperatur, insbesondere von Kleinkindern, gibt es eine Vielzahl von Lösungsvorschlägen, siehe DE-U-298 04 222, US-A-5,559,497, FR-A-26 94 977, WO 90/09570, DD-A-254 643 und GB-A-2 286 684. Die zuletzt genannte Druckschrift offenbart ein medizinisches Thermometer mit den im ersten Teil des Anspruchs 1 angegebenen Merkmalen. Zur Ausführung von Einzelmessungen sind diese Thermometer nicht geeignet, da sie keine Maximumfunktion besitzen.

Häufig wird als Befestigungsort das Handgelenk vorgeschlagen, siehe US-A-5,559,497, FR-A-26 94977 und GB-A-2 286 684. Das Handgelenk ist jedoch als Meßort für kontinuierlich überwachende Körpertemperaturmessungen ungeeignet, da die Meßergebnisse sehr stark von der Umgebungstemperatur und der Abdeckung der Meßstelle (Bekleidung, Bettdecke, Lage des Handgelenks unter dem Körper) abhängen können. Auch die in GB-A-2 286 684 vorgesehene Kalibrierung des Thermometers auf die Körperkerntemperatur durch einen Referenzfühler kann dieses Problem nicht beseitigen, da der Abgleich immer nur für eine konkrete Umgebungssituation gültig ist.

Aus EP-A-0 424 102 ist ein Frauenthermometer zur täglichen Bestimmung der Basaltemperatur bekannt, das als Maximumthermometer arbeitet, für kontinuierlich überwachende Messungen aber ungeeignet ist.

Aus US-A-4,636,093 ist eine Temperaturmeßanordnung bekannt, mit der sich über Strahlungsthermometer mehrere an einem oder mehreren Meßorten erfaßte Temperaturwerte einschließlich zugehöriger Meßzeiten in einem transportablen Aufzeichnungsgerät aufnehmen und anschließend auf eine Verarbeitungseinheit überspielen lassen. Bei den einzelnen Meßwerten, deren jeder einzelne durch manuelle Betätigung von dem Aufzeichnungsgerät aufgenommen wird, kann es sich um Momentanwerte oder innerhalb jeweils einer kurzen Zeitspanne auftretende Maximalwerte handeln.

Aufgabe der Erfindung ist es, ein medizinisches Thermometer zu schaffen, mit dem sowohl kontinuierlich überwachende als auch diskontinuierlich zyklische Messungen als Maximumthermometer ausgeführt werden können.

Gelöst wird diese Aufgabe mit dem in Anspruch 1 angegebenen Thermometer. Danach wird durch das Einstellen der Speicherrate festgelegt, ob das Thermometer die Temperatur der Meßstelle kontinuierlich überwacht oder zyklische bzw. Einzelmessungen ausführt. Es ist somit zum Wechseln der Betriebsart kein zusätzlicher Taster oder Schalter notwendig, der an einem kleinen kompakten Gehäuse nur schwer zu plazieren wäre.

Nach Anspruch 2 wird das Thermometer durch Einstellen der Speicherrate auf Null für diskontinuierliche Messungen konfiguriert. Für jede von Null verschiedene Speicherrate arbeitet das Thermometer nach Anspruch 3 als kontinuierlich messendes Thermometer, das entsprechend der eingestellten Speicherrate regelmäßig die aktuelle Temperatur in einem Datenspeicher ablegt.

Da das Thermometer auch bei kleinen Kindern eingesetzt werden soll, muß ein versehentliches Ausschalten bzw. Umkonfigurieren sicher ausgeschlossen werden. Das gelingt mit dem in Anspruch 4 angegebenen Sicherheitstaster, der beispielsweise einen Ein/Aus-Taster und einen zur Konfiguration notwendige Mode-Taster blockiert. Der Sicherheitstaster ist so im Gehäuse angeordnet, z.B. gegenüber der Gehäuseoberfläche versenkt, daß ein versehentliches Betätigen vermieden wird.

Der nach Anspruch 5 vorgesehene Tageszähler dient dazu, im kontinuierlichen Betrieb den Tag der ununterbrochenen Messung und im diskontinuierlichen Betrieb die Anzahl der gespeicherten Meßwerte anzuzeigen.

Zum Signalisieren von kritischen Abweichungen der gemessenen Temperatur weist die nach Anspruch 6 und 7 vorgesehene akustisch und/oder optisch arbeitende Alarmeinrichtung zwei unabhängig voneinander einstellbare Alarmtemperaturen auf.

Um zu gewährleisten, daß das Thermometer seine Überwachungsfunktion sicher ausführen kann, wird nach Anspruch 8 und 9 in regelmäßigen Abständen die korrekte Funktion des Thermometers durch Messung auf einen Referenzwiderstand selbsttätig getestet und die aktuelle Batteriespannung ermittelt. Abweichungen von den Normwerten werden optisch und/oder akustisch signalisiert.

Die Gestaltung nach Anspruch 10 ist für die Plazierung des Temperaturfühlers sowohl in der Leiste als auch unter der Achsel vorteilhaft. Die gemäß Anspruch 11 vorhandene Aufnahme für verschiedene Befestigungsadapter ermöglicht die Befestigung des Thermometers auf geeignete Weise, z.B. mit Hilfe eines Klettverschlusses.

Das Thermometer ist gemäß Anspruch 12 so leicht und kompakt, daß es problemlos auch von kleinen Kindern längere Zeit am Körper getragen werden kann.

Komfort und Einsatzmöglichkeiten des Thermometers werden durch die Maßnahme des Anspruchs 13 weiter erhöht.

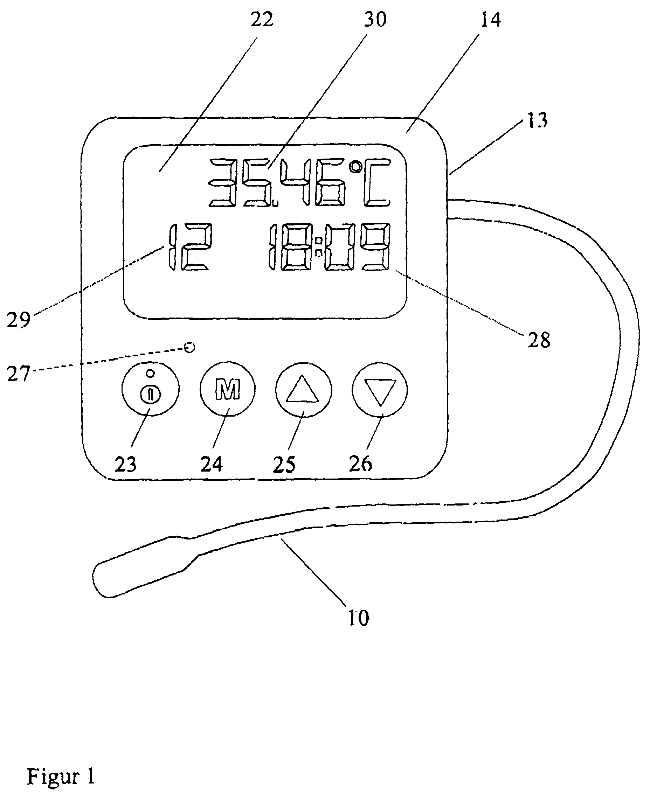

Die Erfindung wird nachfolgend an einem Ausführungsbeispiel anhand von Zeichnungen näher erläutert. Darin zeigt
- Fig. 1: eine Außenansicht,

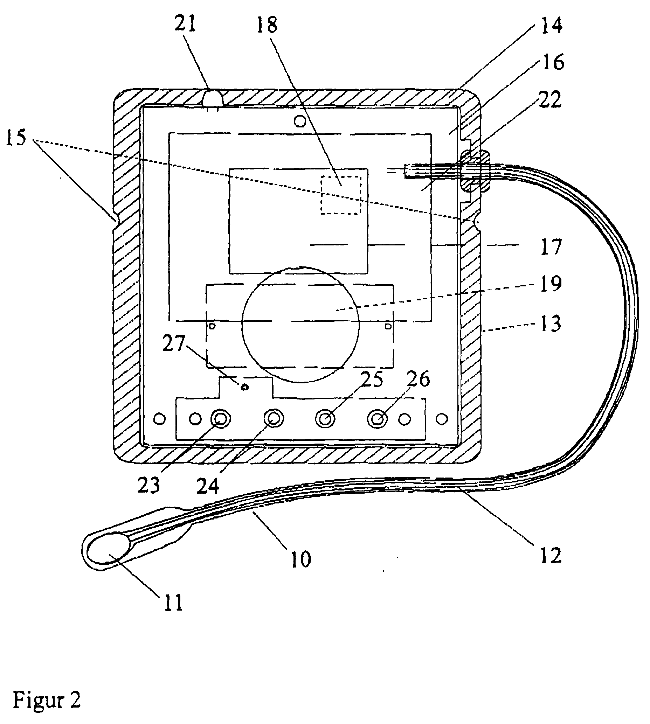

- Fig. 2: einen Schnitt zur Darstellung der prinzipielles Aufbaus des Thermometers, und

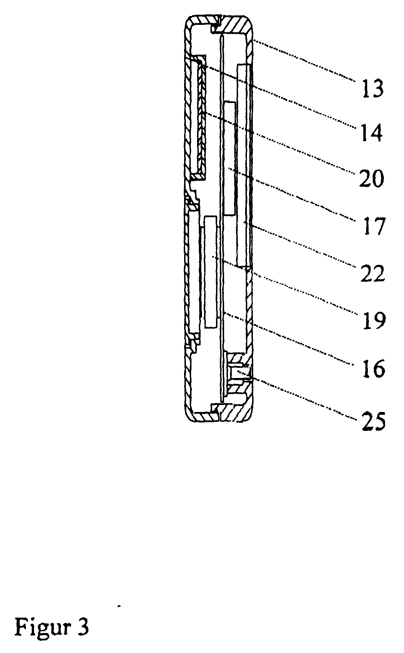

- Fig. 3: einen Längsschnitt durch die Anordnung nach Fig. 2.

Gemäß den Zeichnungen besteht das medizinische Thermometer aus einem Temperaturfühler **10** und einer Meßelektronik **13.** Der aus einem Temperatursensor **11** und einem Kabel **12** gebildete Temperaturfühler **10** ist so gestaltet, daß er Messungen sowohl in der Leiste als auch axillar oder oral gestattet.

Für kontinuierliche Temperaturmessungen bei erkrankten Kleinkindern und Säuglingen wird die Meßelektronik **13** außen am bauchseitigen Rand der Windel befestigt, und der Temperaturfühler **10** wird so in die Windel geschoben, daß er die Temperatur in der Leiste erfassen kann. Zur Befestigung wird aus dem Thermometerzubehör ein geeigneter (nicht dargestellter) Befestigungsadapter mit Klettverschlußstreifen oder Clip ausgewählt und in die am Gehäuse **14** der Meßelektronik **13** vorhandene Adapteraufnahme **15** eingesteckt.

Die Meßelektronik **13** umfaßt auf einer in ihrem Gehäuse **14** vorhandenen Leiterplatte **16** einen Mikrocontroller **17** mit integriertem Datenspeicher **18**, eine Batterie **19**, einen akustischen Signalgeber **20** und eine Schnittstelle **21** zum Anschluß an einen PC oder eine Telemetrieeinrichtung, ferner an der Gehäuseoberseite ein Display **22** mit verschiedenen Anzeigefeldern, von der Oberseite zugängliche Eingabetaster **23** bis **26** und einen versenkt angeordneten Sicherheitstaster **27.**

Eine in dem Mikrocontroller **17** vorhandene Uhr steuert eine Zeitanzeige **28** auf dem Display **22** derart, daß auch bei abgeschaltetem Thermometer die aktuelle Zeit angezeigt wird. Dies erleichtert die Inbetriebnahme des Thermometers, da das Einstellen der Uhrzeit für den Anwender entfällt.

Nach Einschalten mittels des Ein/Aus-Tasters **23** erscheinen auf dem Display **22** außer der Zeitanzeige **28** die aktuelle Temperatur in einer Temperaturanzeige **30** und in einer Tagesanzeige **29** eine den Tag der Messung angebende Zahl.

Das Thermometer beginnt die Messungen mit einer Meßrate von 1/s. Damit ist gewährleistet, daß der Anwender erkennt, daß das Thermometer funktioniert. Nach 15 min Meßzeit wird die Meßrate automatisch auf einen geringeren Wert von 1/min umgestellt, um Energie zu sparen.

Der Mode-Taster **24** dient dazu, verschiedene Einstellebenen auszuwählen. Mit den Auf- und Ab-Tastern **25**, **26** lassen sich in den einzelnen Ebenen Werte verändern bzw. Vorgänge auslösen. Folgende sieben Ebenen sind vorgesehen:
**1**. normaler Meßbetrieb,
**2**. Uhrzeit,
**3**. Alarm 1,
**4**. Alarm 2,
**5**. Speicherrate (Standard 1 h),
**6**. Datenübertragung,
**7**. Clear.

Mit Hilfe der Funktionstaster **24** bis **26** kann die Uhr jederzeit, z.B. nach einem Batteriewechsel, neu gestellt werden. Durch Ausführen des Clear-Befehls wird der Datenspeicher **18** gelöscht und der Tageszähler zurückgesetzt.

Je nach eigenem Ermessen bzw. nach ärztlichem Rat können die Alarmtemperaturen und die Speicherrate des Thermometers eingestellt werden; z.B. untere Alarmtemperatur: 35,0 °C, obere Alarmtemperatur: 37,5 °C, Speicherrate: 1/h. Bei dieser Einstellung wird der Signalgeber **20** aktiviert, wenn die Körpertemperatur des Kindes unter 35 °C absinkt, weil z.B. die Bettdecke das Kind nicht mehr bedeckt. Der Alarm wird auch aktiviert, wenn das Kind das Thermometer durch unkontrollierte Bewegungen von der Meßstelle entfernt. Steigt die Temperatur des Kindes aufgrund einer Erkrankung über 37,5 °C an, so wird ebenfalls der akustische Signalgeber **20** aktiviert.

In Verbindung mit heute weit verbreiteten Raumüberwachungsgeräten für Kleinkinder (Babyphon) ist somit eine Fernüberwachung der Körpertemperatur des Kindes möglich. Die Eltern des Kindes werden akustisch alarmiert, wenn eine gefährliche Situation eintritt. Der Signalgeber **20** kann so beschaffen sein, daß er anstelle eines akustischen Signals oder zusätzlich zu diesem ein optisches Signal erzeugt.

Ein Signal wird auch dann hörbar, wenn die Spannung der Batterie **19** unter einen kritischen Wert absinkt und ein Batteriewechsel notwendig ist, um die Überwachungsfunktion weiter zu gewährleisten.

Bei der gewählten Speicherrate von 1/h wird jede Stunde der letzte ermittelte Temperaturwert gemeinsam mit der Meßzeit und der Tageszahl gespeichert. Die aufgezeichneten Datensätze aus Temperatur, Meßzeit und Tageszahl können mit Hilfe der Auf- und Ab-Taster **25** und **26** jederzeit angezeigt werden.

Es ist ebenfalls möglich, die ermittelten Daten über die Schnittstelle **21**, vorzugsweise als Infrarotschnittstelle ausgebildet, an einen PC zu übertragen. Dort kann die gemessene Temperaturkurve graphisch dargestellt und bei Bedarf z.B. auch per e-Mail an einen Arzt zur Begutachtung weitergeleitet werden.

Der Sicherheitstaster **27** dient dazu, ein versehentliches Ausschalten des Thermometers oder ein unbeabsichtigtes Verstellen z.B. der Alarmtemperaturen zu verhindern. Wird mit dem Taster **27** die Verriegelung aktiviert, so reagiert das Thermometer nicht mehr auf Betätigungen der Taster Ein/Aus **23** und Mode **24**, während die Temperaturanzeige **30** und die Zeitanzeige **28** aktiv bleiben. Auch die gespeicherten Meßwerte können weiterhin durch Drücken der Auf- und Ab-Taster **24**, **25** angezeigt werden.

Durch Fieber ausgelöste Krämpfe gibt es nicht nur bei Säuglingen, sondern auch bei Kindern bis zu sechs und mehr Lebensjahren. Da diese Krämpfe zu beliebigen Zeitpunkten auftreten können, ist eine ständige Körpertemperaturüberwachung notwendig.

Bei windeltragenden Kindern wird das Thermometer wie beschrieben angewendet. Bei größeren Kindern kann das Thermometer mit dem Clip außen an der Hose befestigt und der Fühler in der Leiste plaziert werden. Eine andere Variante besteht darin, das Thermometer in einem Holster am Brustkorb zu tragen. Dies ermöglicht eine Plazierung des Temperaturfühlers **10** in der Achsel. Dort wird der Fühler mit einem Spezialpflaster (mit guter Hautverträglichkeit des Klebers) fixiert.

Bei plötzlich auftretendem Temperaturanstieg mit Überschreitung der eingestellten Alarmtemperatur wird der akustische Alarm ausgelöst. Das Kind selbst bzw. eine andere in der Nähe befindliche Person kann geeignete Maßnahmen einleiten, um den Fieberkrampf zu verhindern bzw. um qualifizierte Hilfe anzufordern (Notarzt).

Ein bei älteren, pflegebedürftigen Personen häufig auftretendes Problem besteht in Störungen der körpereigenen Temperaturregulierung. Es kann zu lebensbedrohlichen Auskühlungen kommen. Um diesem Fall vorzubeugen, kann mit Hilfe des Thermometers die Körpertemperatur laufend kontrolliert werden. Die Signalübertragung zum Pflegepersonal kann mit Hilfe eines Raumüberwachungsgerätes erfolgen, wie es bei Kleinkindern eingesetzt wird. Geeignete Meßorte sind die Leiste bzw. unter der Achsel.

Durch Einstellen der Speicherrate auf Null wird das Thermometer von einem kontinuierlich messenden Überwachungsthermometer zum Maximumthermometer umgestellt. Nun kann das Thermometer z.B. zur Aufzeichnung der Basaltemperatur verwendet werden. In diesem Betriebsmodus werden als Besonderheit die eingestellten Alarmwerte nicht überwacht. Werden mehrere Messungen an einem Tag durchgeführt, so wird stets nur die Messung mit dem höchsten Temperaturwert gespeichert. Auf diese Weise ist es möglich, eine Messung mit fehlerhaftem Ergebnis zu wiederholen, ohne daß die Temperaturkurve durch Doppelmessungen am gleichen Tag verfälscht wird. Der ermittelte Temperaturwert wird gemeinsam mit der Uhrzeit und der Tageszahl gespeichert. Die Auswertung der gemessenen Temperaturkurve kann sowohl manuell durch simples Abschreiben der Meßergebnisse und Eintragen in eine Tabelle als auch komfortabel durch Übertragung der Meßwerte auf einen PC erfolgen.

Auch als ganz gewöhnliches Fieberthermometer ist das medizinische Thermometer mit einer eingestellten Speicherrate von Null verwendbar.

Mögliche Anwendungsgebiete für das medizinische Thermometer bestehen im Krankenhaus bei der Säuglingsüberwachung oder der postoperativen Patientenüberwachung.

## Patentansprüche

1. Medizinisches Thermometer mit einem Temperaturfühler (**10**) und einer Meßelektronik (**13**) mit Mikrocontroller (**17**) und Datenspeicher (**18**) zur periodischen Aufzeichnung des von dem Temperaturfühler (**10**) gemessenen Temperaturwertes, und mehreren Tastern (**23**...**27**) zur Beeinflussung der Arbeitsweise des Mikrocontrollers (**17**),
dadurch **gekennzeichnet**, daß die Speicherrate des Datenspeichers (**18**) zwischen kontinuierlichem und diskontinuierlichem Betrieb umschaltbar ist.

2. Thermometer nach Anspruch 1, wobei für diskontinuierliche Messungen eine Speicherrate von Null einstellbar ist.

3. Thermometer nach Anspruch 1 oder 2, wobei es bei diskontinuierlichen Messungen als Maximumthermometer arbeitet.

4. Thermometer nach einem der vorhergehenden Ansprüche mit einem Sicherheitstaster (**27**) zum Blockieren eines Ein/Aus- und des Mode-Tasters (**23, 24**).

5. Thermometer nach einem der vorhergehenden Ansprüche mit einer Tagesanzeige (**29**).

6. Thermometer nach einem der vorhergehenden Ansprüche mit einem Signalgeber (**20**), der durch zwei unabhängig voneinander einstellbare Alarmtemperaturen aktivierbar ist.

7. Thermometer nach Anspruch 6, wobei der Signalgeber (**19**) akustisch und/oder optisch arbeitet.

8. Thermometer nach Anspruch 6 oder 7, wobei der Mikrocontroller (**17**) selbsttätig periodische Referenzmessungen auslöst und auswertet und bei Abweichungen von einem Normwert den Signalgeber (**20**) betätigt.

9. Thermometer nach einem der Ansprüche 6 bis 8, wobei der Mikrocontroller (**17**) bei Absinken der Versorgungsspannung unter einen kritischen Wert den Signalgeber (**20**) betätigt.

10. Thermometer nach einem der vorhergehenden Ansprüche, wobei der Temperatursensor (**10**) in ein an ein Gehäuse (**14**) der Meßelektronik (**13**) anschließbares wasserdichtes Kabel (**11**) integriert ist.

11. Thermometer nach einem der vorhergehenden Ansprüche mit einer an einem Gehäuse (**14**) der Meßelektronik (**13**) vorgesehenen Adapteraufnahme (**15**) für mindestens einen Befestigungsadapter.

12. Thermometer nach einem der vorhergehenden Ansprüche in einer auch für Kleinkinder tragbaren kompakten Bauform.

13. Thermometer nach einem der vorhergehenden Ansprüche mit einer Schnittstelle (**21**) zur Fernübertragung des Datenspeicherinhalts.
